# EUROPÄISCHE PATENTSCHRIFT

(11) **EP 2 753 433 B1**
(45) Veröffentlichungstag und Bekanntmachung des Hinweises auf die Patenterteilung: **04.10.2017**
(21) Anmeldenummer: 12740604.9
(22) Anmeldetag: 30.07.2012
(51) Int. Cl.: A61M 15/08, B05B 11/00, A61M 11/00, G01F 11/02, B05B 1/34

(54) **FLÜSSIGKEITSSPENDER**
LIQUID DISPENSER
DISTRIBUTEUR DE LIQUIDE

(30) Priorität: 09.09.2011 DE 102011082420
(43) Veröffentlichungstag der Anmeldung: 16.07.2014
(73) Patentinhaber: Aptar Radolfzell GmbH, 78315 Radolfzell (DE)
(72) Erfinder: AUERBACH, Judith, 9052 Niederteufen (CH); GREINER-PERTH, Jürgen, 78244 Gottmadingen (DE)
(74) Vertreter: Patentanwaltskanzlei Cartagena
(86) Internationale Anmeldenummer: PCT/EP2012/064899
(87) Internationale Veröffentlichungsnummer: WO 2013/034365

(56) Entgegenhaltungen:
- WO-A2-2005/048875
- FR-A1- 2 862 107
- JP-U- 62 139 971
- US-A1- 2003 057 297

## Beschreibung

### Anwendungsgebiet und Stand der Technik

Die Erfindung betrifft einen Flüssigkeitsspender nach dem Oberbegriff des Anspruchs 7 und dessen Austragkopf nach dem Oberbegriff des Anspruchs 1.

Ähnliche Flüssigkeitsspender sind aus dem Stand der Technik allgemein bekannt. So beschreiben beispielsweise die EP 1 768 789 B1 und die WO 2010/004224 A2 ähnliche Spender. Solche Spender dienen dem Austrag von Flüssigkeiten, wie beispielsweise von pharmazeutischen oder kosmetischen Flüssigkeiten. Sie sind als tragbare Einheiten ausgestaltet, die ein Patient und Nutzer problemlos mit sich führen kann.

Aus der WO 2006/042641 A1 ist ein Flüssigkeitsspender bekannt, dessen Dosiervolumen über ein Schieberventil bestimmt wird, welches durch eine ringförmige Dosierkammer umgeben ist. Dabei dient das Schieberventil selbst nicht unmittelbar der Druckbeaufschlagung der Flüssigkeit, sondern schließt lediglich während der Betätigung zweitweise den Flüssigkeitspfad zwischen Dosierkammer und einem Flüssigkeitsspeicher, um dadurch mittelbar die Druckerhöhung in der Dosierkammer und den Austrag der Flüssigkeit aus dieser zu steuern.

Aus der FR 2862107 A1 ist ein Spender bekannt, der über einen Kolben verfügt, der in einer unteren Endlage bereits in einem zylindrischen Dosierraum angeordnet ist, so dass mit Beginn einer Betätigung unmittelbar ein Austragvorgang beginnt.

Weiterer Stand der Technik, der den technologischen Hintergrund zur vorliegenden Erfindung darstellt, ist aus den Dokumenten US 2003/0057297 A1, WO 2005/048875 A2 und JP S62139971 U bekannt.

Insbesondere bei solchen Spendern, die dem Austrag pharmazeutischer Flüssigkeiten dienen, kommt es abhängig vom konkreten Anwendungszweck zum Teil auf hohe Genauigkeit im Hinblick auf die ausgetragene Flüssigkeitsmenge an. Gleichzeitig müssen gattungsgemäße Spender, die üblicherweise nicht zur Wiederverwendung ausgebildet sind, preisgünstig herstellbar sein.

### Aufgabe und Lösung

Aufgabe der Erfindung ist es, einen gattungsgemäßen Spender und einen gattungsgemäßen Austragkopf zur Verfügung zu stellen, die eine hohe Dosiergenauigkeit bieten. Dabei sollten dieser Spender insbesondere und dieser Austragkopf auch preisgünstig herstellbar und montierbar sein.

Erfindungsgemäß wird hierzu ein gattungsgemäßer Austragkopf vorgeschlagen, der nach Anspruch 1 ausgestaltet ist. Weiterhin wird ein erfindungsgemäßer Flüssigkeitsspender nach Anspruch 7 vorgeschlagen.

Die Fördereinrichtung eines gattungsgemäßen Austragkopfes oder eines Flüssigkeitsspenders mit einem solchen Austragkopf verfügt somit über den genannten zylindrischen Dosierraum, der im Zuge einer Betätigung des Spenders vom dabei an den Wandungen des Dosierraums anliegenden Kolben durchquert wird. Während der Kolben sich im Dosierraum befindet und somit an der zylindrischen Wandung des Dosierraums anliegt, drückt er die Flüssigkeit, die sich zuvor im Dosierraum befand, in Richtung der Austrittsseite des Dosierraums und somit in Richtung der Austragöffnung.

Durch die Aufweitung des Dosierraums sowohl an dessen Eingangsseite als auch an dessen Ausgangsseite wird eine hohe Genauigkeit beim Austrag erzielt. Dies liegt darin begründet, dass bei der Betätigung der Fördereinrichtung ein Austrag so lange nicht erfolgt, so lange der Kolben noch in der Eintrittskammer angeordnet ist und somit die Dosierkammer auf ihrer Eintrittsseite noch nicht dicht verschließt, und dass der während der Betätigung aufgebaute Druck in der Flüssigkeit unmittelbar in jenem Augenblick zusammenbricht, in dem der Kolben an der Austrittsseite des Dosierraums den Kontakt zur Wandung des Dosierraums wieder verliert. Die für den Austrag der Flüssigkeit genutzte Teilstrecke der Bewegung des Kolbens gegenüber dem Dosierraum von der Ausgangslage bis zur Endlage ist demnach die Strecke zwischen einer ersten Relativposition, in der der Kolben erstmals mit der Dosierraumwandung in Kontakt gerät, und einer zweiten Relativposition, in der dieser Kontakt wieder verloren geht.

Der lineare Zusammenhang zwischen dieser für den Austrag wirksamen Strecke und der ausgetragenen Flüssigkeitsmenge ermöglicht eine besonders einfache Anpassung des Dosiervolumens eines Flüssigkeitsspenders. Zum Zwecke dieser Anpassung ist es lediglich erforderlich, die Länge des Dosierraums zu verändern, wobei dies in der Regel nur die Anpassung des die Dosierraumwandung bildenden Bauteils erforderlich macht, während alle anderen Bauteile des Flüssigkeitsspenders für verschiedene gewünschte Dosiervolumina identisch bleiben können.

Dadurch, dass bei der erfindungsgemäßen Gestaltung der den beidseitig aufgeweiteten Dosierraum durchquerende Kolben selbst der Druckbeaufschlagung der Flüssigkeit dient, wird verglichen mit der oben genannten WO 2006/042641 A1 eine recht schlanke Bauweise des erfindungsgemäßen Spenders möglich.

Vorzugsweise verfügt der Austragkopf bzw. der Flüssigkeitsspender mit einem solchen Austragkopf über zwei gegeneinander bewegliche und durch einen Benutzer unmittelbar handhabbare Gehäuseabschnitte, wobei an einem ersten der Gehäuseabschnitte der Dosierraum ortsfest vorgesehen ist und wobei an einem zweiten der Gehäuseabschnitte der Kolben ortsfest vorgesehen ist. Somit entspricht die von außen manuell verursachte Bewegung der Gehäuseabschnitte auch der Bewegung des Kolbens gegenüber dem Dosierraum. Auf komplizierte mechanische Kopplungen zwischen der Bewegung des Kolbens und des Dosierraums einerseits und der von außen zugänglichen Gehäuseabschnitte kann verzichtet werden.

Erfindungsgemäß ist vorgesehen, dass der Flüssigkeitsspeicher bzw. der Ankopplungsabschnitt des Austragkopfes für den Flüssigkeitsspeicher mit der Fördereinrichtung über zwei unterschiedliche Flüssigkeitspfade verbunden ist. Ein erster dieser Flüssigkeitspfade führt vom Flüssigkeitsspeicher außen am Kolben vorbei in die Eintrittskammer oder die Austrittskammer. Dieser erste Flüssigkeitspfad ist jener Flüssigkeitspfad, der während einer Betätigung der Fördereinrichtung verschlossen wird, wenn der Kolben in den Dosierraum einfährt, und der geöffnet wird, wenn der Kolben am gegenüberliegenden Ende wieder aus dem Dosierraum austritt. In diesem Flüssigkeitspfad ist zwischen dem Flüssigkeitsspeicher und dem Kolben vorzugsweise kein Ventil vorgesehen, so dass zu jenem Zeitpunkt, zu dem der Kolben den Dosierraum an der Austrittsseite verlässt, eine Egalisierung des Drucks im Flüssigkeitsspender bis hin zum Flüssigkeitsspeicher stattfindet.

Vorgesehen ist weiterhin, dass ein zweiter Flüssigkeitspfad vorgesehen ist, der vom Flüssigkeitsspeicher bzw. vom Ankopplungsabschnitt des Austragkopfes für den Flüssigkeitsspeicher durch den Kolben hindurch in den Dosierraum führt, wobei ein bei Überdruck im Dosierraum schließendes Einlassventil in diesem Flüssigkeitspfad vorgesehen ist. Dieser zweite Flüssigkeitspfad führt somit nicht am Kolben vorbei, sondern durch den Kolben hindurch. Da dieser zweite Flüssigkeitspfad somit nicht unmittelbar durch das Eindringen des Kolbens in den Dosierraum verschlossen wird, weist er ein Rückschlagventil auf, welches bei Druckbeaufschlagung der Flüssigkeit im Dosierraum schließt. Dieser zweite Flüssigkeitspfad gestattet es, dass während eines Rückhubs, bei dem der Kolben und der Dosierraum in Richtung ihrer Ausgangslage gegeneinander bewegt werden, ein Einsaugen neuer Flüssigkeit aus dem Flüssigkeitsspeicher in den Dosierraum bereits stattfinden kann, während der Kolben noch im Dosierraum angeordnet ist.

Zur Erleichterung des Eintretens des Kolbens in den Dosierraum ist es vorzugsweise vorgesehen, dass in einem Übergangsbereich zwischen der Eintrittskammer und dem Dosierraum eine umlaufende Fase vorgesehen ist. Diese schließt vorzugsweise einen Winkel zwischen 15° und 45° mit der Betätigungsrichtung ein. Durch eine hier vorgesehene Phase kann der Entstehung einer Beschädigung an dem Kolben bzw. an dessen Kolbenlippe entgegengewirkt werden. Auch ist es zur Erleichterung des Eintretens des Kolbens in den Dosierraum möglich, die Aufweitung der Eintrittskammer und/oder der Austrittskammer dadurch zu erzielen, dass lediglich in Teilbereichen des Umfangs eine Aufweitung gegeben ist. Dies könnte zum Beispiel durch längserstreckte Nuten realisiert werden, die in der Wandung der Eintrittskammer oder Austrittskammer vorgesehen sind, welche im Übrigen den gleichen Innendurchmesser wie die Dosierkammer aufweist.

Eine besonders bevorzugte Ausgestaltung sieht vor, dass der Dosierraum ortsfest an einem Gehäuseabschnitt des Gehäuses vorgesehen ist, wobei dieser Gehäuseabschnitt mindestens zwei miteinander ortsfest verbundene Bauteile aufweist, von denen ein Außenbauteil von außen zugänglich ist und von denen ein Innenbauteil die Dosierkammer aufweist. Die zweiteilige Gestaltung des den Dosierraum aufweisenden Gehäuseabschnitts führt zu einer sehr einfachen modularen Anpassbarkeit des Spenders an verschiedene Anwendungszwecke und insbesondere an verschiedene gewünschte Dosiervolumina. So kann das vorzugsweise hülsenähnlich aufgebaute Außenbauteil, welches zur Aufnahme des vorzugsweise ebenfalls vorzugsweise etwa hülsenförmig aufgebauten Innenbauteils ausgebildet ist, unverändert bleiben, wenn das Innenbauteil zur Erzielung eines geänderten Dosiervolumens hinsichtlich der Länge seiner Dosierkammer angepasst wird. Eine geringere Teilevielfalt und somit verringerte Herstellungskosten sind die Folge.

Ein erfindungsgemäßer Flüssigkeitsspender weist vorzugsweise eine zwischen seinen beiden Gehäuseabschnitten angeordnete Rückstellfeder auf, durch die die Gehäuseabschnitte in Richtung der Ausgangslage kraftbeaufschlagt werden. Bei einer besonders bevorzugten Gestaltung ist diese Rückstellfeder derart angeordnet, dass das Innenbauteil durch sie permanent gegen das Außenbauteil gedrückt wird. Die gegenüberliegende Seite der Rückstellfeder stützt sich üblicherweise am anderen Gehäuseabschnitt mit dem daran vorgesehenen Kolben ab. Durch die permanente Kraftbeaufschlagung des Innenbauteils gegen das Außenbauteil kann auf eine weitergehende und mit Aufwand verbundene Kopplungseinrichtung zwischen den beiden Bauteilen verzichtet werden. Die Relativbeweglichkeit des Außenbauteils gegenüber dem anderen Gehäuseabschnitt mit dem daran vorgesehenen Kolben ist vorzugsweise durch entsprechende Anschläge begrenzt, die damit auch der Trennbarkeit entgegenwirken.

Insbesondere von Vorteil ist es, wenn der Austragöffnung ein druckabhängig öffnendes Auslassventil zugeordnet ist. Dieses Auslassventil gewährleistet einen Mindestdruck der Flüssigkeit beim Austrag, beispielsweise zur Erzeugung eines Sprühstrahls. Es gewährleistet auch, dass vor Eintreten des Kolbens in den Dosierraum kein Medium austreten kann, da der geringe Überdruck, der durch die Bewegung des Kolbens in der Eintrittskammer noch vor Eintreten in die Dosierkammer bewirkt wird, nicht ausreicht, um das Auslassventil zu öffnen. Das Ventil bewirkt darüber hinaus, dass während des Rückhubs die Austragöffnung verschlossen ist, so dass durch sie hindurch keine Luft in den Spender eingesogen werden kann.

Im Sinne einer besonders einfachen Bauform ist es weiterhin von Vorteil, wenn ein einstückiges Bauteil am Spender vorgesehen ist, welches einerseits zur Befestigung an einem Auslassstutzen eines flaschenartig ausgebildeten Flüssigkeitsspeichers ausgebildet ist und an welchem andererseits der Kolben vorgesehen ist.

### Kurzbeschreibung der Zeichnungen

Weitere Aspekte und Vorteile der Erfindung ergeben sich außer aus den Ansprüchen aus der nachfolgenden Beschreibung eines bevorzugten Ausführungsbeispiels der Erfindung, welches anhand der Figuren nachfolgend erläutert wird. Dabei zeigen:
- Fig. 1: einen erfindungsgemäßen Flüssigkeitsspender in einer Gesamtdarstellung und
- Fig. 2a bis 2c: einen Austragkopf des Flüssigkeitsspenders der Fig. 1 in drei Stadien während der Betätigung.

### Detaillierte Beschreibung des Ausführungsbeispiels

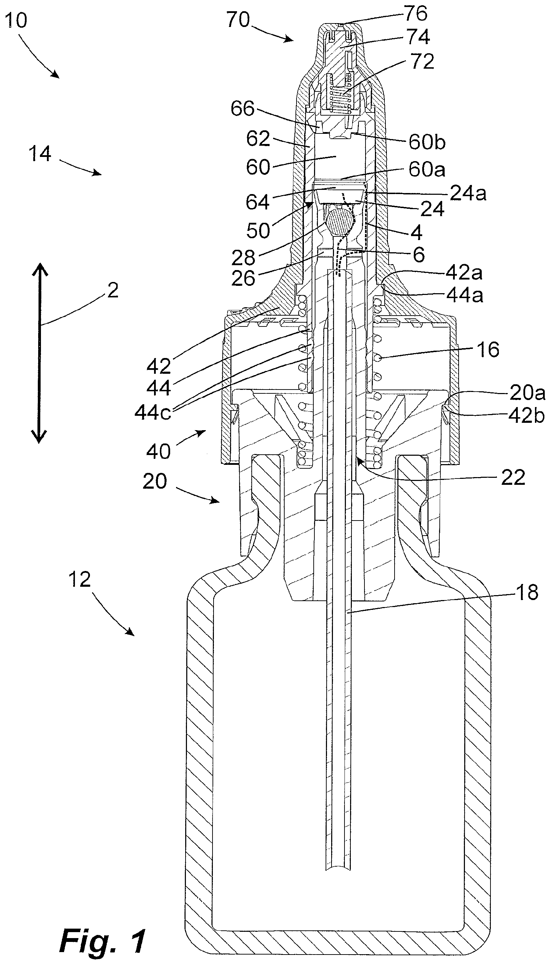

Fig. 1 zeigt einen erfindungsgemäßen Spender mit einem flaschenartigen Flüssigkeitsspeicher 12 sowie einen daran angebrachten Austragkopf 14. Der Austragkopf 14 umfasst als Hauptbestandteile einen zur Anbringung am Flüssigkeitsspeicher 12 vorgesehenen ersten Gehäuseabschnitt 20 sowie einen demgegenüber in Richtung einer Betätigungsrichtung 2 relativbeweglichen zweiten Gehäuseabschnitt 40. Zwischen den Gehäuseabschnitten 20, 40 ist eine Rückstellfeder 16 vorgesehen, die den zweiten Gehäuseabschnitt 40 permanent in Richtung seiner in Fig. 1 dargestellten oberen Endposition kraftbeaufschlagt.

Gemeinsam bilden der erste Gehäuseabschnitt 20 und der zweite Gehäuseabschnitt 40 eine Fördereinrichtung 50 nachfolgend beschriebener Art. Der einstückig ausgebildete Gehäuseabschnitt 20 weist zentrisch einen sich nach oben erstreckenden und in Art eines Hohlrohrs ausgebildeten Fortsatz 22 auf, der an seinem oberen Ende in einen Kolben 24 mit einer umlaufenden Kolbenlippe 24a übergeht. Innerhalb dieses Fortsatzes 22 ist ein Steigrohr 18 vorgesehen, durch welches hindurch Flüssigkeit aus dem Flüssigkeitsspeicher 12 bis zum oberen Ende des Fortsatzes 22 gelangen kann. Dort kann die Flüssigkeit entlang eines ersten Flüssigkeitspfades 4 durch Radialkanäle 26 seitlich aus dem Fortsatz 22 austreten. Sie kann jedoch auch durch ein als Rückschlagventil ausgebildetes Einlassventil 28 entlang eines zweiten Flüssigkeitspfades 6 stirnseitig und damit innenseitig der Kolbenlippe 24a aus dem Fortsatz 22 austreten.

Der Fortsatz 22 erstreckt sich in den zweiten Gehäuseabschnitt 40 hinein, der insbesondere ein Außenbauteil 42 und ein Innenbauteil 44 aufweist. Das Innenbauteil 44 ist rohrartig oder hülsenartig ausgebildet und in das hülsenartig ausgebildete Außenbauteil 42 eingesetzt. Durch korrespondierende Anschläge 44a, 42a an den beiden Bauteilen 42, 44 wird dabei die Relativposition definiert. Einer besonderen Form der Festlegung des Innenbauteils 44 im Außenbauteil 42 bedarf es nicht, da das Innenbauteil 44 durch die Rückstellfeder 16 permanent gegen das Außenbauteil 42 gedrückt wird. Das Außenbauteil 42 wiederum ist durch einen nach innen gewandten Haltekragen 42b am ersten Gehäuseabschnitt 20 hinsichtlich seiner Beweglichkeit gegenüber dem ersten Gehäuseabschnitt 20 limitiert. Das Innenbauteil 44 dichtet zusammen mit einem zylindrischen Bereich des Fortsatz 22 flüssigkeitsführende Bereich des Spenders Austragkopfes 14 gegenüber der Umgebung ab und weist hierfür am unteren Ende nach innen gewandte angeformte Dichtringe 44c auf.

Zum Zusammenwirken mit dem Kolben 24 ist am zweiten Gehäuseabschnitt 40 und zwar an dessen Innenbauteil 44 ein Dosierraum 60 vorgesehen. Dieser Dosierraum 60 ist dadurch definiert, dass er von einer umlaufenden Wandung 62 umgeben wird, die derart auf den Kolben 24 abgestimmt ist, dass bei an der Dosierkammerwandung 62 anliegender Kolbenlippe 24a die Flüssigkeit aus dem Dosierraum 60 nicht mehr entlang des ersten Flüssigkeitspfades 4 zurück in Richtung des Flüssigkeitsspeichers 12 gelangen kann. An der Eingangsseite 60a der Dosierkammer schließt sich eine Eintrittskammer 64 mit aufgeweitetem Querschnitt an. An der Austrittsseite 60b der Dosierkammer 60 schließt sich eine ebenfalls aufgeweitete Austrittskammer 66 an.

Sowohl bei Anordnung des Kolbens 24 und insbesondere seiner Kolbenlippe 24a in der Eintrittskammer 64 als auch in der Austrittskammer 66 liegt die Kolbenlippe 24a zumindest nicht umlaufend abdichtend an der Innenwandung des Innenbauteils 44 an. Eine vollständige Trennung des Drucks oberhalb des Kolbens 24 vom Druck im Flüssigkeitsspender 12 ist daher nur dann gegeben, wenn der Kolben 24 sich innerhalb des Dosierraums 60 befindet und seine Kolbenlippe 24a an der Dosierkammerwandung 62 anliegt. Sowohl vor dem Eintreten des Kolbens 24 in die Dosierkammer 60 von unten als auch nach dem Austreten des Kolbens 24 aus der Dosierkammer 60 nach oben besteht dagegen eine kommunizierende Verbindung zwischen allen flüssigkeitsführenden Bereichen innerhalb des Spenders.

Jenseits der Austrittskammer 66 schließt sich ein druckabhängiges öffnendes Auslassventil 70 an, welches über eine Feder 72 verfügt, durch die ein Ventilkörper 74 permanent gegen eine Austragöffnung 76 gedrückt wird. Lediglich bei ausreichendem Überdruck kann dieser Ventilkörper 74 gegen die Kraft der Feder 72 nach unten verlagert werden, so dass er die Austragöffnung 76 zum Zwecke des Flüssigkeitsaustritts freigibt.

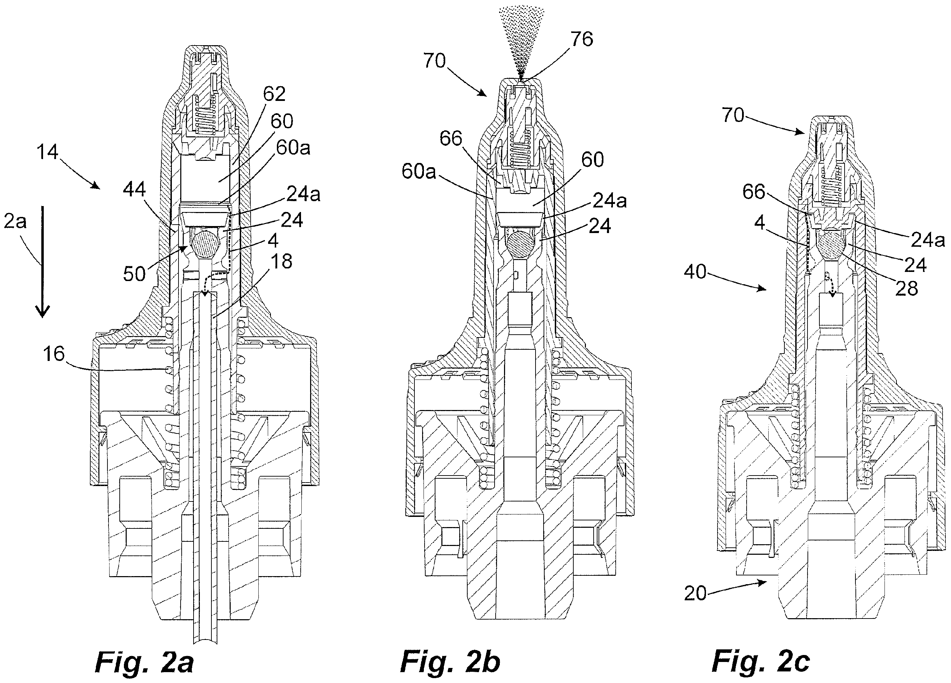

Die Fig. 2a bis 2c verdeutlichen einen Austrittsvorgang mit dem Flüssigkeitsspender der Fig. 1. Dabei ist der Spender als bereits betriebsfertig anzusehen. Es sind also alle bestimmungsgemäß flüssigkeitsführenden Räume im Bereich der Fördereinrichtung 50 bereits mit Flüssigkeit befüllt. Auf die Herstellung des betriebsfertigen Zustandes wird nachfolgend noch eingegangen.

Fig. 2a zeigt den Austragkopf 14 des Spenders der Fig. 1 in einer Ausgangslage, in der der zweite Gehäuseabschnitt 40 gegenüber dem flüssigkeitsspeicherfesten ersten Gehäuseabschnitt 20 seine obere Endlage einnimmt. Ausgehend von diesem Ausgangszustand erfolgt eine Betätigung, indem der zweite Gehäuseabschnitt 40 in Richtung des Pfeils 2a gegen die Kraft der Rückstellfeder 16 hinabgedrückt wird. Dieses Hinabdrücken, welches mit einer Verlagerung des Kolbens 24 gegenüber dem Innenbauteil 44 einhergeht, führt zu einer Reduzierung des flüssigkeitsführenden Volumens im Bereich der Fördereinrichtung 50. Überschüssige Flüssigkeit aus der Fördereinrichtung wird entlang des ersten Flüssigkeitspfades 4 zurück in das Steigrohr 18 gedrückt. Diese erste Phase der Betätigung endet, wenn die Kolbenlippe 24a des Kolbens von der Eintrittsseite 60a aus in die Dosierkammer 60 eintritt. Hierbei legt sich die Kolbenlippe 24a an die Dosierkammerwandung 62 an und verschließt somit den ersten Flüssigkeitspfad 4. Der zweite Flüssigkeitspfad 6 durch das Einlassventil 28 hindurch wird ebenfalls spätestens in diesem Augenblick unter dem Eindruck des sich in der Druckkammer 60 einstellenden Überdrucks geschlossen.

Mit jenem Augenblick, in dem die Kolbenlippe 24a umlaufend zur Anlage an der Dosierkammerwandung 62 kommt, beginnt eine zweite Phase der Betätigung, die durch Fig. 2b verdeutlicht wird. Das fortgesetzte Hinabdrücken des zweiten Gehäuseabschnitts 40 führt zu einer Druckbeaufschlagung der Flüssigkeit in der Dosierkammer 60. Da diese Flüssigkeit nicht mehr in Richtung des Flüssigkeitsspeichers 12 entweichen kann, bewirkt der sich aufbauende Druck ein Öffnen des Auslassventils 70, so dass die Flüssigkeit aus der Dosierkammer 60 und anderen flüssigkeitsführenden Bereichen im Bereich des Auslassventils 70 durch die Austragöffnung 76 hindurch nach außen gelangen kann.

Der Austragvorgang endet in jenem Augenblick, in welchem die Dichtlippe 24a in die Austrittskammer 66 eintritt. Hier verliert die Dichtlippe 24a zumindest partiell den Kontakt mit der Wandung 62, so dass der erste Flüssigkeitspfad 4 in der in Fig. 2c verdeutlichten Art und Weise wieder geöffnet ist. Schlagartig bricht der Druck in der Austrittskammer 66 und den flüssigkeitsführenden Bereichen des Auslassventils 70 zusammen, so dass das Auslassventil 70 unmittelbar schließt und eine etwaige Fortsetzung der Relativbewegung zwischen den Gehäuseabschnitten 20, 40 lediglich noch ein Entweichen der Flüssigkeit aus diesen Bereichen in das Steigrohr 18 verursacht.

Während des nachfolgenden Rückhubs gelangt der Kolben 24 wieder in den Dosierraum 60 und verschließt dabei wieder den ersten Flüssigkeitspfad 4. Der sich während des Rückhubs im Dosierraum 60 einstellende Unterdruck führt allerdings zu einem Öffnen des Einlassventils 28, so dass während des Rückhubs Flüssigkeit entlang des zweiten Flüssigkeitspfades 6 aus dem Flüssigkeitsspeicher 12 durch das Steigrohr 18 hindurch in den Dosierraum 60 eingesogen wird. Sobald der Rückhub beendet ist und somit wieder der Zustand der Fig. 2a erreicht wurde, ist der Spender für einen erneuten Austrag vorbereitet.

Vor der erstmaligen Inbetriebnahme des Spenders 10 sind die Dosierkammer 60, die Eintrittskammer 64 und die Austrittskammer 66 und die Freiräume im Bereich des Ventils 70 noch mit Luft befüllt. Der ersten zwei, drei Hübe führen zu einem Herausdrücken dieser Luft. Diese wird im Rahmen dieser sogenannten Priming-Hübe soweit komprimiert, dass das Auslassventil 70 öffnet und die Luft entweichen lässt.

Der dargestellte Spender und insbesondere der dargestellte Austragkopf 14 gestattet bei sehr einfachem Aufbau und sehr geringer Teilezahl ein präzises Austragen vorgegebener Flüssigkeitsmengen. Dabei ist die im Rahmen einer Betätigungsbewegung auszutragende Flüssigkeit alleine durch das Zylindervolumen des Dosierraums 60 definiert. Eine Anpassung des Spenders an abweichende Dosiervolumina ist daher sehr einfach möglich. Bei dem vorliegenden Spender reicht es aus, das Innenbauteil 44 gegen ein leicht modifiziertes Innenbauteil 44 auszutauschen, dessen Dosierraum durch eine Verlagerung des Übergangsbereichs zwischen der Eintrittskammer und dem Dosierraum verändert ist. Eine Anpassung anderer Bauteile des Spenders ist nicht erforderlich.

## Patentansprüche

1. Austragkopf (14) für einen Flüssigkeitsspender (10), insbesondere für pharmazeutische Flüssigkeiten, mit
- einem Gehäuse (20, 40), mit einem Ankopplungsabschnitt zur Anbringung an einem Flüssigkeitsspeicher (12),
- einer Austragöffnung (76) und
- einer manuell betätigbaren Fördereinrichtung (50) zur Förderung von Flüssigkeit vom Flüssigkeitsspeicher (12) zur Austragöffnung (76),
wobei
die Fördereinrichtung (50) einen Dosierraum (60) mit zylindrischer Wandung (62) und einen hinsichtlich des Querschnitts zur gleitenden Bewegung an der zylindrischen Wandung (62) des Dosierraums (60) angepassten Kolben (24) umfasst, wobei der Dosierraum (60) und der Kolben (24) manuell zwischen einer Ausgangslage und einer Endlage gegeneinander verlagerbar sind, wobei
- der Dosierraum (60) eine Austrittsseite (60b) aufweist, durch die er mit der Austragöffnung (76) verbunden ist, und eine Eintrittsseite (60a) aufweist, durch die er mit dem Flüssigkeitsspeicher (12) verbunden ist,
- sich an die Austrittsseite (60b) des Dosierraums (60) eine Austrittskammer (66) und an die Eintrittsseite (60a) des Dosierraums (60) eine Eintrittskammer (64) anschließt, deren Querschnitt jeweils größer ist als der Querschnitt des Kolbens (24), und
- der Kolben (24) in der Ausgangslage in der Eintrittskammer (64) angeordnet ist und in der Endlage in der Austrittskammer (66) angeordnet ist und bei der Überführung von der Ausgangslage in die Endlage die Flüssigkeit im Dosierraum (60) druckbeaufschlagt zum Zwecke des Austrags von Flüssigkeit durch die Austragöffnung (76) hindurch,
**dadurch gekennzeichnet, dass**
der Ankopplungsabschnitt für den Flüssigkeitsspeicher (12) mit der Fördereinrichtung über zwei Flüssigkeitspfade (4, 6) verbunden ist, wobei
- ein erster Flüssigkeitspfad (4) vom Ankopplungsabschnitt für den Flüssigkeitsspeicher (12) am Kolben (24) vorbei in die Eintrittskammer (64) oder die Austrittskammer (66) führt, wobei dieser erste Flüssigkeitspfad (4) durch den Kolben (24) verschlossen wird, wenn der Kolben (24) an der Eintrittsseite (60a) in den Dosierraum (60) einfährt, und geöffnet wird, wenn der Kolben (24) an der Austrittsseite (60b) aus dem Dosierraum (60) austritt,
und
- ein zweiter Flüssigkeitspfad (6) vom Ankopplungsabschnitt für den Flüssigkeitsspeicher (12) durch den Kolben (24) hindurch in den Dosierraum (60) führt, wobei ein bei Überdruck im Dosierraum (60) schließendes Einlassventil (28) im zweiten Flüssigkeitspfad vorgesehen ist.

2. Austragkopf (14) nach Anspruch 1,
**dadurch gekennzeichnet, dass**
das Gehäuse zwei gegeneinander bewegliche Gehäuseabschnitte (20, 40) aufweist, wobei an einem ersten der Gehäuseabschnitte (40) der Dosierraum (60) ortsfest vorgesehen ist und wobei an einem zweiten der Gehäuseabschnitt (20) der Kolben (24) ortsfest vorgesehen ist.

3. Austragkopf (14) nach einem der vorstehenden Ansprüche,
**dadurch gekennzeichnet, dass**
in einem Übergangsbereich zwischen der Eintrittskammer (64) und der Dosierkammer (60) eine Fase vorgesehen ist, vorzugsweise mit einem Winkel zwischen 15° und 45° gegenüber der Betätigungsrichtung (2).

4. Austragkopf (14) nach einem der vorstehenden Ansprüche,
**dadurch gekennzeichnet, dass**
der Dosierraum (60) ortsfest an einem Gehäuseabschnitt (40) des Gehäuses vorgesehen ist, wobei dieser Gehäuseabschnitt (40) mindestens zwei miteinander ortsfest verbundene Bauteile (42, 44) aufweist, von denen ein Außenbauteil (42) von außen zugänglich ist und von denen ein Innenbauteil (44) die Dosierkammer (60) aufweist.

5. Austragkopf (14) nach Anspruch 4,
**dadurch gekennzeichnet, dass**
der Flüssigkeitsspeicher (12) eine Rückstellfeder (16) zur Überführung der Gehäuseabschnitte (20, 40) aus der Endlage in die Ausgangslage aufweist, wobei durch diese Rückstellfeder (16) das Innenbauteil (44) permanent gegen das Außenbauteil (42) gedrückt wird.

6. Austragkopf (14) nach einem der vorstehenden Ansprüche,
**dadurch gekennzeichnet, dass**
der Austragöffnung (76) ein druckabhängig öffnendes Auslassventil (70) zugeordnet ist.

7. Flüssigkeitsspender (10), insbesondere für pharmazeutische Flüssigkeiten, mit einem Flüssigkeitsspeicher (12) zur Aufnahme der Flüssigkeit vor dem Austrag und einem Austragkopf (14),
**dadurch gekennzeichnet, dass**
der Austragkopf (14) nach einem der vorstehenden Ansprüche ausgebildet ist.

8. Flüssigkeitsspender (10) nach Anspruch 7,
**dadurch gekennzeichnet, dass**
der Flüssigkeitsspeicher (12) flaschenartig mit einem Auslassstutzen ausgebildet ist, wobei ein einstückiges Bauteil (20) vorgesehen ist, welches zur Befestigung an diesem Auslassstutzen vorgesehen ist und an welchem Kolben (24) vorgesehen ist.

## Claims

1. Discharge head (14) for a liquid dispenser (10), in particular for pharmaceutical liquids, with
- a housing (20, 40) having a coupling portion for mounting on a liquid reservoir (12),
- a discharge opening (76), and
- a manually actuatable conveyor device (50) for conveying liquid from the liquid reservoir (12) to the discharge opening (76),
wherein
the conveyor device (50) comprises a metering chamber (60) with a cylindrical wall (62), and a piston (24) that is adapted, in terms of the cross section, to the cylindrical wall (62) of the metering chamber (60) so as to allow a sliding movement thereon, wherein the metering chamber (60) and the piston (24) are able to be manually displaced, relative to each other, between a starting position and an end position, wherein
- the metering chamber (60) has an outlet side (60b) through which it is connected to the discharge opening (76), and an inlet side (60a) through which it is connected to the liquid reservoir (12),
- the outlet side (60b) of the metering chamber (60) is adjoined by an outlet chamber (66), and the inlet side (60a) of the metering chamber (60) is adjoined by an inlet chamber (64), of which the cross section is in each case larger than the cross section of the piston (24), and
- the piston (24) is arranged in the inlet chamber (64) when in the starting position and is arranged in the outlet chamber (66) when in the end position, and, during the transition from the starting position to the end position, the liquid in the metering chamber (60) is pressurized for the purpose of discharging liquid through the discharge opening (76),
**characterized in that** the coupling portion for the liquid reservoir (12) is connected to the conveyor device via two liquid paths (4, 6), wherein
- a first liquid path (4) leads from the coupling portion for the liquid reservoir, past the piston (24) and into the inlet chamber (64) or the outlet chamber (66), wherein this first liquid path (4) is closed by the piston (24) when the piston (24) enters the metering chamber (60) at the inlet side (60a), and is opened when the piston (24) emerges at the outlet side (60b) from the metering chamber (60), and
- a second liquid path (6) leads from the coupling portion for the liquid reservoir (12), through the piston (24) and into the metering chamber (60), wherein an inlet valve (28) that closes when there is an overpressure in the metering chamber (60) is provided in the second liquid path.

2. Discharge head (14) according to Claim 1, **characterized in that** the housing has two housing portions (20, 40) movable relative to each other, wherein the metering chamber (60) is provided in a fixed position on a first of the housing portions (40), and wherein the piston (24) is provided in a fixed position on a second of the housing portions (20).

3. Discharge head (14) according to one of the preceding claims, **characterized in that** a bevel, preferably with an angle of between 15° and 45° in relation to the actuation direction (2), is provided in a transition area between the inlet chamber (64) and the metering chamber (60).

4. Discharge head (14) according to one of the preceding claims, **characterized in that** the metering chamber (60) is provided in a fixed position on a housing portion (40) of the housing, wherein this housing portion (40) has at least two component parts (42, 44) connected to each other in a fixed position, of which an outer component part (42) is accessible from the outside, and of which an inner component part (44) has the metering chamber (60).

5. Discharge head (14) according to Claim 4, **characterized in that** the liquid reservoir (12) has a restoring spring (16) for transferring the housing portions (20, 40) from the end position to the starting position, wherein the inner component part (44) is pressed permanently against the outer component part (42) by this restoring spring (16).

6. Discharge head (14) according to one of the preceding claims, **characterized in that** the discharge opening (76) is assigned an outlet valve (70) that opens in a pressure-dependent manner.

7. Liquid dispenser (10), in particular for pharmaceutical liquids, with a liquid reservoir (12) for receiving the liquid prior to the discharge and a discharge head (14), **characterized in that** the discharge head (14) is configured according to one of the preceding claims.

8. Liquid dispenser (10) according to Claim 7, **characterized in that** the liquid reservoir (12) is designed like a bottle with an outlet nozzle, wherein a one-piece component part (20) is provided, which is intended to be secured on this outlet nozzle and on which the piston (24) is provided.

## Revendications

1. Tête de distribution (14) pour un distributeur de liquide (10), en particulier pour des liquides pharmaceutiques, avec
- un boîtier (20, 40), avec une partie de couplage pour le placement sur un réservoir de liquide (12),
- un orifice de distribution (76) et
- un dispositif de transport actionnable manuellement (50) pour le transport de liquide du réservoir de liquide (12) à l'orifice de distribution (76),
dans laquelle le dispositif de transport (50) comprend une chambre de dosage (60) avec une paroi cylindrique (62) et un piston (24) adapté en section transversale en vue d'un mouvement de glissement à la paroi cylindrique (62) de la chambre de dosage (60), dans laquelle la chambre de dosage (60) et le piston (24) sont déplaçables manuellement l'un par rapport à l'autre entre une position initiale et une position finale, dans laquelle
- la chambre de dosage (60) présente un côté de sortie (60b), par lequel elle est reliée à l'orifice de distribution (76), et présente un côté d'entrée (60a), par lequel elle est reliée au réservoir de liquide (12),
- une chambre de sortie (66) se raccorde au côté de sortie (60b) de la chambre de dosage (60) et une chambre d'entrée (64) se raccorde au côté d'entrée (60a) de la chambre de dosage (60), dont la section transversale respective est plus grande que la section transversale du piston (24), et
- le piston (24) est disposé dans la position initiale dans la chambre d'entrée (64) et est disposé dans la position finale dans la chambre de sortie (66) et lors du transfert de la position initiale à la position finale il applique une pression au liquide dans la chambre de dosage (60) en vue de la distribution de liquide à travers l'orifice de distribution (76),
**caractérisée en ce que** la partie de couplage pour le réservoir de liquide (12) est reliée au dispositif de transport par deux chemins de liquide (4, 6), dans laquelle
- un premier chemin de liquide (4) conduit de la partie de couplage pour le réservoir de liquide (12) le long du piston (24) jusque dans la chambre d'entrée (64) ou la chambre de sortie (66), dans laquelle ce premier chemin de liquide (4) est fermé par le piston (24), lorsque le piston (24) entre dans la chambre de dosage (60) sur le côté d'entrée (60a) et est ouvert, lorsque le piston (24) sort de la chambre de dosage (60) sur le côté de sortie (60b),
et
- un deuxième chemin de liquide (6) conduit de la partie de couplage pour le réservoir de liquide (12) à travers le piston (24) jusque dans la chambre de dosage (60), dans laquelle il est prévu dans le deuxième chemin de liquide une soupape d'admission (28) qui se ferme en cas de surpression dans la chambre de dosage (60).

2. Tête de distribution (14) selon la revendication 1, **caractérisée en ce que** le boîtier présente deux parties de boîtier (20, 40) mobiles l'une par rapport à l'autre, dans laquelle la chambre de dosage (60) est prévue sous forme fixe sur une première des parties de boîtier (40) et dans laquelle le piston (24) est prévu sous forme fixe sur une deuxième des parties de boîtier (20).

3. Tête de distribution (14) selon l'une quelconque des revendications précédentes, **caractérisée en ce qu'**il est prévu un chanfrein dans une région de transition entre la chambre d'entrée (64) et la chambre de dosage (60), de préférence avec un angle compris entre 15° et 45° par rapport à la direction d'actionnement (2).

4. Tête de distribution (14) selon l'une quelconque des revendications précédentes, **caractérisée en ce que** la chambre de dosage (60) est prévue sous forme fixe sur une partie de boîtier (40) du boîtier, dans laquelle cette partie de boîtier (40) présente au moins deux composants assemblés fixement l'un à l'autre (42, 44), dont un composant extérieur (42) est accessible de l'extérieur et dont un composant intérieur (44) présente la chambre de dosage (60).

5. Tête de distribution (14) selon la revendication 4, **caractérisée en ce que** le réservoir de liquide (12) présente un ressort de rappel (16) pour le transfert des parties de boîtier (20, 40) de la position finale à la position initiale, dans laquelle le composant intérieur (44) est en permanence pressé contre le composant extérieur (42) par ce ressort de rappel (16).

6. Tête de distribution (14) selon l'une quelconque des revendications précédentes, **caractérisée en ce qu'**une soupape d'échappement (70) s'ouvrant en fonction de la pression est associée à l'orifice de distribution (76).

7. Distributeur de liquide (10), en particulier pour des liquides pharmaceutiques, avec un réservoir de liquide (12) destiné à contenir le liquide avant la distribution et une tête de distribution (14), **caractérisé en ce que** la tête de distribution (14) est réalisée selon l'une quelconque des revendications précédentes.

8. Distributeur de liquide (10) selon la revendication 7, **caractérisé en ce que** le réservoir de liquide (12) est réalisé en forme de flacon avec un embout de sortie, dans lequel il est prévu un composant en une pièce (20), qui est prévu pour la fixation à cet embout de sortie et sur lequel est prévu le piston (24).
